# EUROPÄISCHE PATENTANMELDUNG

(11) **EP 2 016 959 A1**
(43) Veröffentlichungstag der Anmeldung: **21.01.2009**
(21) Anmeldenummer: 08158414.6
(22) Anmeldetag: 17.06.2008
(51) Int. Cl.: A61L 31/08, A61L 31/14, A61L 31/16

(54) **Stent mit einer Beschichtung**

(30) Priorität: 13.07.2007 DE 102007032686
(71) Anmelder: BIOTRONIK VI Patent AG, 6341 Baar (CH)
(72) Erfinder: Wittchow, Eric, 90403 Nürnberg (DE)
(74) Vertreter: Lindner-Vogt, Karin L.

(57) **Zusammenfassung**

Die Erfindung betrifft einen Stent aus einem metallischen Grundkörper und mit einer selenhaltigen Beschichtung oder Füllung einer Kavität.

## Beschreibung

Die Erfindung betrifft einen Stent aus einem metallischen Grundkörper und mit einer Beschichtung.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper, durch den der Blutfluss ungehindert fließen kann. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatwerkstoffe in bioaktive, bioinerte und degradierbare/resorbierbare Werkstoffe unterteilt werden.

Für die Zwecke der vorliegenden Erfindung sind lediglich metallische Implantatwerkstoffe für Stents von Interesse. Biokompatible Metalle und Metalllegierungen für Permanentimplantate umfassen rostfreie Stähle (z. B. 316L), Kobaltbasislegierungen (z. B. CoCr (L605), CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf metallische Elemente hängt ab von der Konzentration, Einwirkdauer und Art der Zuführung. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität und damit eine Verbesserung der Restenoserate erreichen lässt, wenn metallische Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen.

Trotz der erreichten Fortschritte besteht noch immer ein hohes Bedürfnis, eine bessere Integration des Stents in sein biologisches Umfeld zu erreichen und dadurch die Restenoserate zu senken.

### Erfindungsgemäße Lösung

Nach einem ersten Aspekt der Erfindung werden eine oder mehrere der oben geschilderten Probleme durch einen Stent aus einem metallischen Grundkörper und mit einer selenhaltigen Beschichtung oder Füllung einer Kavität gelöst.

Der Erfindung liegt die Erkenntnis zugrunde, dass in einem gesunden Organismus ein Gleichgewicht aus Zellvermehrung (Proliferation) und Zellsterben (Apoptose) besteht. Kommt es zu einer Restenose nach Stentimplantation ist das Gleichgewicht beider Prozesse gestört und die Proliferation gewinnt überhand über den natürlichen Zelltod. Die bisherigen Strategien zur Vermeidung der Restenose setzen auf eine Hemmung der Proliferation. Porcine histologische Präparate stenosierter Gefäße konnten jedoch keine erhöhte Konzentration an Proliferationsmarkern gegenüber dem umliegenden Gewebe belegen. Bauriedel (J. Vasc. Res. 2004; 41 (6); 525-534) untersuchte atherektomische Proben von Patienten mit symptomatischer In-Stent Restenose immunohistologisch und konnte zeigen, dass der programmierte Zelltod (Apoptose) in älteren Läsionen signifikant verringert ist, verglichen mit primären Atheromen. Dies stützt die Annahme, dass die Apoptose weniger effektiv erfolgt als in gesundem Gewebe. Hier setzt die Erfindung an: Das Ungleichgewicht von Zellvermehrung und Zelltod soll durch Steigerung der Apoptoserate ausgeglichen werden. Der Vorteil gegenüber der Proliferationshemmung, die gleichermaßen auf unerwünschte neointimale Zellen wie auch auf die notwendigen Endothelzellen wirkt, ist das ungehinderte Einwachsen des Stents. Wird die Vermehrung von Endothelzellen gestört, verzögert sich die Abdeckung des Stents mit Gewebe und es kommt zu vermehrter Thrombenbildung und der Gefahr eines tödlichen Gefäßverschlusses.

Es hat sich gezeigt, dass der Einsatz elementaren Selens oder von Selenverbindungen als Bestandteil einer Beschichtung oder Füllung einer Kavität eines metallischen Stents zur Steigerung der Apoptose führt. Der den positiven Einfluss von Selen auf die Apoptose zugrunde liegende Wirkmechanismus ist weitgehend noch ungeklärt. Vermutlich wird das direkt den apoptotischen Vorgang auslösende Enzym Caspase-3 aktiviert.

Vorzugsweise werden anorganische Selenverbindungen verwendet, insbesondere Selendioxid (SeO₂), Selendisulfid (SeS₂), Selenide (besonders bevorzugt MgSe), Selenite, Selenate oder Selenophosphate (H₃SePO₄). Anorganische Selenverbindungen sind gegenüber organischen Selenverbindungen thermisch zumeist stabiler, so dass die Herstellung und Sterilisation des Stents vereinfacht ist. Dennoch können auch organische Selenverbindungen wie Selen-ocystein, Selenodiglutathion, Selenomethionin und andere Selenoproteine Einsatz finden.

Das die Apoptose stimulierende Material kann Bestandteil einer Beschichtung sein oder die Beschichtung besteht gänzlich aus dem Material. In ersterem Fall kann zum Beispiel ein Selensalz in pulverisierter Form in eine biodegradierbare polymere Matrix eingebettet sein. Außerdem kann es Bestandteil des Elektrolyten bei der Herstellung einer Magnesium-Konversionsschicht (MAGOXID, MAGPASS; BIOXID) auf einem aus einer biokorrodierbaren Magnesiumlegierung bestehenden Stent sein, wodurch es in die Konversionsschicht eingelagert wird und bei deren Degradation freigesetzt wird. Die Beschichtung wird in der Regel unmittelbar auf dem Grundkörper des Stents aufgebracht; gegebenenfalls können jedoch Zwischenschichten vorhanden sein. Alternativ kann das die die Apoptose stimulierende Material Bestandteil einer Kavitätenfüllung sein. Die Kavität befindet sich in der Regel an der Oberfläche des Stents; bei Stents mit einem biodegradierbaren Grundkörper kann die Kavität auch im Innern des Grundkörpers angeordnet sein, so dass die Freisetzung des Materials erst nach Freilegung erfolgt. Vorzugsweise enthält die Beschichtung oder Füllung 0,1 bis 20 µg freies oder gebundenes Selen pro 1 mm Stentlänge.

Vorzugsweise enthält die Beschichtung oder Füllung zusätzlich Arsen oder eine arsenhaltige Verbindung. Es hat sich gezeigt, dass eine Kombination von Selen und Arsen die Toxizität senkt, so dass unerwünschte Nebenreaktionen gemindert werden. Selen wirkt sich demnach positiv auf die arseninduzierte Zyto-toxizität und Beeinflussung der Zellviabilität aus. Dadurch kann die Bioverträglichkeit von Arsen verbessert werden, einem weiteren die Apoptose fördernden Element.

Nach einer weiteren bevorzugten Ausführungsform weist der metallische Grundkörper eine poröse Oberfläche auf, die mit der selenhaltigen Beschichtung bedeckt ist. Mit anderen Worten, die zugänglichen Poren der porösen Oberfläche des metallischen Grundkörpers sind mit der selenhaltigen Beschichtung bedeckt/befüllt. Hierdurch kann die Kontaktfläche zum biologischen System vergrößert werden und es können die erfindungsgemäß gewünschten Effekte auf die Apoptose verstärkt werden.

Vorzugsweise ist das metallische Grundgerüst aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung. Die genannten biokorrodierbaren metallischen Werkstoffe sind zumindest weitgehend chemisch inert gegenüber Selen und Selenverbindungen, so dass ein negativer Einfluss auf die Degradation des Stents nicht zu erwarten ist.

Besonders bevorzugt ist eine Kombination, bei der das metallische Grundgerüst des Stents aus einer biodegradierbaren Magnesiumlegierung besteht und die Beschichtung MgSe enthält oder aus MgSe besteht.

Als biokorrodierbar (oder biodegradierbar) im Sinne der Erfindung werden Legierungen und Elemente bezeichnet, bei denen in physiologischer Umgebung ein Abbau/Umbau stattfindet, so dass der aus dem Werkstoff bestehende Teil des Implantats ganz oder zumindest überwiegend nicht mehr vorhanden ist.

Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl2 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Nachfolgend wird die Erfindung anhand von Ausführungsbeispielen näher erläutert.

Ein Stent aus der biodegradierbaren Magnesiumlegierung WE43 (nach ASTM) wurde entfettet und getrocknet. Der Stent kann an seiner Oberfläche Kavitäten aufweisen. Die Beschichtung wurde wie folgt durchgeführt:

### Beispiel 1: Beschichtung des Stents mit wirkstoffhaltiger Polymermatrix

Es wird eine 0,05 bis 0,4%ige Lösung eines Poly(orthoester) in trockenem THF bereitgestellt, der aus 3,9 Diethyliden-2,4,8,10-tetraoxaspiro[5.5] undecan und trans-Cyclohexandimethanol, 1,6-Hexandiol, Triethylenglycol und Triethylenglycolglycolid (molares Verhältnis: 15/40/40/5) hergestellt wurde. Der Stent wird von Staub und Rückständen gereinigt und in eine geeignete Stentbeschichtungsapparatur eingespannt. Es wird eine klare, 10%ige Lösung aus Selenomethionin in THF derart zu der Polymerlösung zugegeben, dass Polymer und Wirkstoff ein Gewichts-Verhältnis von 30/70 bis 80/20 haben (bevorzugt: 60/40). Mit Hilfe eines Airbrush Systems wird der sich drehende Stent unter konstanten Umgebungsbedingungen (Raumtemperatur; 42% Luftfeuchte) halbseitig beschichtet. Bei einem Düsenabstand von 20 mm ist ein 18 mm langer Stent nach ca. 10 min beschichtet. Die Schichtmasse ist so zu wählen, dass der Stent 0,1 µg - 10 µg (bevorzugt 1µg) Selenmethionin/mm enthält. Nach Erreichen der beabsichtigten Schichtmasse wird der Stent 5 min bei Raumtemperatur getrocknet, ehe nach Drehen des Stents und erneutem Einspannen die unbeschichtete Seite auf dieselbe Weise beschichtet wird. Der fertig beschichtete Stent wird für 24h bei 80°C in einem Vakuumofen getrocknet.

### Beispiel 2: Beschichtung eines mit Kavitäten versehenen Stents mit elementarem Selen

Man stellt eine gesättigte ethanolische Lösung aus pulvriger seleniger Säure (H₂SeO₃) her und hängt bei Raumtemperatur den mit Kavitäten versehenen Stent an einer geeigneten Vorrichtung für 3-5 min derart in diese Lösung, dass der Stent von allen Seiten benetzt wird. Die Aufhängung des Stents besteht zweckmäßigerweise aus einem Magnesiumdraht, da andere edlere Metalle mit Magnesium ein Lokalelement ausbilden würden und Kunststoffe häufig nicht die Temperaturen für den darauf folgenden Sinterschritt aushalten. Der an der Oberfläche rötlich gefärbte Stent wird aus der Lösung genommen und vorsichtig mit Druckluft abgeblasen. Der Stent wird an demselben Magnesiumdraht für 2 min in einen 230°C heißen Glühofen unter Luftatmosphäre gehängt, worauf das rötliche Selen schmilzt, teilweise in die Kavitäten eindringt und nach Erstarren bei Raumtemperatur einen dünnen metallischen Film ausbildet. Gravimetrisch kann die Menge an biologisch aktiver Substanz bestimmt werden. Die Abgabe von Selen kann durch das Aufbringen eines polymeren Deckschicht modifiziert werden.

### Beispiel 3: Modifizierung eines Stents mit selenhaltiger Konversionsschicht

Der Stent wird für 1 min in einer gesättigten Lösung aus KOH in Isopropanol gereinigt und kurz mit viel deionisiertem Wasser abgespült. Anschließend erfolgt eine anodische Oxidation in einem wässerigen Elektrolytbad, welches 30 g/l H₂SeO₃ (selenige Säure), 55 g/l H₃PO₄ (Phosphorsäure) und 300 g/l Hexamethylentetramin enthält. Der pH-Wert ist mit NH₄OH auf 8,5 eingestellt. Die anodische Oxidation erfolgt bei 20°C mit einer Stromdichte von 1,1 A/dm² und einer bis zu 240 V aufsteigenden Spannung mit gepulsten Gleichstrom für 5 min. Die erhaltene selenhaltige Schichtdicke beträgt 5 µm.

## Patentansprüche

1. Stent aus einem metallischen Grundkörper und mit einer selenhaltigen Beschichtung oder Füllung einer Kavität.

2. Stent nach Anspruch 1, bei dem die Beschichtung oder Füllung elementares Selen, SeO₂, SeS₂, Selenide, Selenite, Selenate oder Selenophosphate enthält.

3. Stent nach Anspruch 1 oder 2, bei dem das metallische Grundgerüst des Stents aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung besteht.

4. Stent nach Anspruch 3, bei dem das metallische Grundgerüst des Stents aus einer biodegradierbaren Magnesiumlegierung besteht und die Beschichtung MgSe enthält oder aus MgSe besteht.

5. Stent nach einem der vorhergehenden Ansprüche, bei dem die Beschichtung oder Füllung 0,1 bis 20 µg freies oder gebundenes Selen pro 1 mm Stentlänge enthält.

6. Stent nach einem der vorhergehenden Ansprüche, bei dem die Beschichtung oder Füllung zusätzlich Arsen oder eine arsenhaltige Verbindung enthält.
